# EUROPÄISCHE PATENTSCHRIFT

(11) **EP 2 825 150 B1**
(45) Veröffentlichungstag und Bekanntmachung des Hinweises auf die Patenterteilung: **22.08.2018**
(21) Anmeldenummer: 13707372.2
(22) Anmeldetag: 01.03.2013
(51) Int. Cl.: A61K 8/35, A61Q 5/06

(54) **MITTEL ZUM FÄRBEN UND/ODER MATTIEREN VON KERATINHALTIGEN FASERN ENTHALTEND NEUARTIGE 1,4-DIAMINOANTHRACHINONFARBSTOFFE**
MEANS FOR DYEING AND/OR MATTING KERATINIC FIBRES CONTAINING NOVEL 1,4 DIAMINO-ANTHRAQUINONE DYES
AGENTS POUR TEINDRE ET/OU MATER DES FIBRES KÉRATINIQUES, CONTENANT DE NOUVEAUX COLORANTS DE 1,4-DIAMINOANTHRAQUINONE

(30) Priorität: 14.03.2012 DE 102012203981
(43) Veröffentlichungstag der Anmeldung: 21.01.2015
(73) Patentinhaber: Henkel AG & Co. KGaA, 40589 Düsseldorf (DE)
(72) Erfinder: GEBERT, Antje, 40545 Düsseldorf (DE); KROOS, Astrid, 40789 Monheim (DE); NEMITZ, Ralph, 41363 Jüchen (DE)
(86) Internationale Anmeldenummer: PCT/EP2013/054178
(87) Internationale Veröffentlichungsnummer: WO 2013/135503

(56) Entgegenhaltungen:
- EP-A2- 2 263 642
- WO-A1-95/13813
- WO-A2-2011/160864
- FR-A- 1 326 406
- GB-A- 1 053 300
- GB-A- 2 004 293
- JP-A- S57 193 430
- US-A- 3 576 587
- US-A- 3 597 254
- US-A- 4 310 666
- TAYLOR R F ET AL: "High-performance liquid chromatography of cancer chemotherapeutic agents: bis(substituted aminoalkylamino)anthraquinones", JOURNAL OF CHROMATOGRAPHY, ELSEVIER SCIENCE PUBLISHERS B.V, NL, vol. 187, no. 1, 4 January 1980 (1980-01-04), pages 212-217, XP026483561, ISSN: 0021-9673, DOI: 10.1016/S0021-9673(00)87888-7 [retrieved on 1980-01-04]

## Beschreibung

Die vorliegende Erfindung betrifft Mittel zum Färben und/oder Mattieren von keratinhaltigen Fasern, insbesondere menschlichen Haaren, die neuartige Derivate des 1,4-Diaminoanthrachinons enthalten. Weiterhin betrifft die Erfindung die Verwendung dieser Mittel sowie ein entsprechendes Verfahren.

Für das Färben von keratinischen Fasern kommen im allgemeinen entweder direktziehende Farbstoffe oder Oxidationsfarbstoffe zur Anwendung. Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Des Weiteren können einige Oxidationsfarbstoffvorprodukte bzw. bestimmte Mischungen von Oxidationsfarbstoffvorprodukten bei Personen mit empfindlicher Haut sensibilisierend wirken. Direktziehende Farbstoffe werden unter schonenderen Bedingungen appliziert. Ihr Nachteil liegt jedoch darin, dass die Färbungen häufig nur über unzureichende Echtheitseigenschaften verfügen, insbesondere bei der Haarwäsche, aber auch gegenüber äußeren Einflüssen, wie Sonnenlicht oder reaktiven Umweltchemikalien, wie beispielsweise Schwimmbadwasser.

Eine besondere Herausforderung für die Haarfärbung mit direktziehenden Farbstoffen stellt die gleichmäßige Färbung von häufig vorbehandeltem Haar, wie beispielsweise gebleichtem oder dauergewelltem Haar, dar, bei dem die Faser in den verschiedenen Längen oder verschieden behandelten Bereichen eine sehr unterschiedlich starke Vorschädigung besitzt. Während der Färbung selbst kann das Färbemittel auf unterschiedlich vorgeschädigtem Haar ein ungleichmäßiges Färbeverhalten zeigen, aber auch durch wiederholte Haarwäsche können die Farbstoffe in den unterschiedlichen Haarbereichen verschieden stark auswaschen werden, wodurch ein uneinheitliches und damit unerwünschtes Farbergebnis erzielt wird.

Eine erste Aufgabe der vorliegenden Anmeldung ist es daher, Färbemittel für keratinische Fasern, insbesondere menschliche Haare, bereitzustellen, die hinsichtlich der Farbtiefe und der Echtheitseigenschaften, wie insbesondere Licht-, Reib- und Waschechtheit sowie Schweiß- und Kaltwellechtheit, gute anwendungstechnische Eigenschaften besitzen. Schließlich ist besonders wünschenswert, Färbemittel mit gutem Egalisiervermögen bereitzustellen.

Sollen keratinische Fasern oxidativ aufgehellt oder blondiert werden, können direktziehende Farbstoffte auch in Kombination mit Oxidationsmitteln eingesetzt werden. Zum Blondieren von Haaren werden in der Regel wässrige Wasserstoffperoxid-Lösungen - entweder allein oder in Kombination mit weiteren, als Bleichaktivatoren wirkenden Oxidationsmitteln wie beispielsweise Persulfatsalzen - auf die Keratinfasern aufgebracht. Um eine ausreichende Blondierwirkung zu erzielen, sind derartige Mittel üblicherweise stark alkalisch eingestellt, der pH-Wert liegt dabei in der Regel zwischen 9 und 10,5. Durch die Einwirkung der Oxidationsmittel werden die Melanine, die natürlichen, farbgebenden

Pigmente der Haarfaser, oxidativ zerstört und auf diese Weise eine Entfärbung bzw. Aufhellung der Fasern erreicht. Die Melanine sind im Cortex der Haarfaser lokalisiert und lassen sich in zwei Pigmentklassen einordnen. Eumelanine stellen die erste, bräunlich-schwarzen Pigmentklasse dar, während die rötlichen, schwefelreicheren Pigmente als Pheomelanine bezeichnet werden. Aufgrund der unterschiedlichen Resistenzen der verschiedenen Pigmenttypen gegenüber Oxidationsmitteln werden die Phäo- und Eumelanine jedoch nicht immer gleichmäßig entfärbt. Zudem kann in dunkleren Haaren mit hohem Melaningehalt der Abbau der Melanine nur teilweise oder unvollständig erfolgen, so dass nach der Blondierung ein Restanteil der farbgebenden Pigmente im Haar verbleibt. In diesen Fällen führt der Restgehalt der nach dem oxidativen Prozess im Haar noch vorhandenen Melanine zu einer gelblichen bis rötlichen Nuancenverschiebung. Daher kommt es insbesondere beim Blondieren von dunkeren Haaren zu einer Farbverschiebung in Richtung warmer Töne. Üblicherweise sind solche Farbverschiebungen in Richtung warmer Töne beim Anwender unerwünscht. Daher wird dieser Farbverschiebung zumeist durch eine Tönung in der entsprechenden Komplementärfarbe gemäß der Farbenlehre entgegengewirkt. Ziel ist dabei ein silbrig-kühler Eindruck des Bleicherbebnisses. Der Fachmann spricht in diesem Zusammenhang von einer Mattierung. Zur Mattierung von orange-stichigen Blondnuancen können insbesondere blaue, direktziehende Farbstoffe eingesetzt werden. Für die möglichst vollständige Abschwächung des orangen Farbeindrucks ist es hierbei von Vorteil, wenn der blaue Farbstoff selbst keinen Rotanteil in seiner Färbung besitzt. Farbstoffe in reinen Blau- und insbesondere in gräulichen Blautönen sind im Vergleich zu violettstichigen Blaufarbstoffen daher besser zur Mattierung eines zu orangen Blondierergebnisses geeignet. Trotz der Vielzahl der in Haarfärbeprodukten einsetzbaren Blaufarbstoffe sind aus dem Stand der Technik jedoch keine Farbstoffe bekannt, die alle vorgenannten Voraussetzungen in optimaler Weise erfüllen. Daher besteht nach wie vor großer Bedarf an neuartigen Direktziehern mit ensprechenden färbenden und mattierenden Eigenschaften.

Somit war es insbesondere die Aufgabe der vorliegenden Erfindung, neuartige Mattierungsfarbstoffe zu finden, welche die üblichen, an direktziehende Farbstoffe gestellten Echtheitsanforderungen erfüllen, eine gute Lagerstabilität besitzen und darüber hinaus einen blauen, stark ins gräuliche changierenden Farbton ohne Rotanteil besitzen. Die Farbstoffe sollen in Form einer Nachbehandlung nach dem oxidativen Blondierprozess, bei einstufigen Verfahren aber auch gleichzeitig mit dem Oxidationsmittel angewendet werden können, weshalb sie zusätzlich eine gute Stabilität gegenüber den Oxidationsmitteln aufweisen müssen. Im Rahmen ihrer Untersuchungen hat die Anmelderin nun überraschend gefunden, dass die in spezieller Form substituierten 1,4-Diaminoanthrachionderviate der allgemeinen Formel (I) die zuvor beschriebenen Anforderungen an neuartige Mattierungsfarbstoffe in optimaler Weise erfüllen. Die GB 1 053 300 beschreibt einen Prozess zum Färben von Haaren, bei welchem 1,4-Diaminoanthrachinonderivate eingesetzt werden können, die an ihren Aminogruppen einen oder zwei Dialkylaminoalkyl-Substituenten tragen. Diese Farbstoffe sollen ein gutes Färbevermögen, eine hohe Wasserlöslichkeit und hohe Lichtbeständigkeit besitzen. Das Nuancenspektrum reicht von intensiv blau über blauviolett bis zu mauve, pink und blau-grün. Graustichige Blaufärbungen können mittels dieser Farbstoffe jedoch nicht erzielt werden. Die DE 1 644 306 offenbart ein Verfahren zur Herstellung von 1,4-Diaminoanthrachinonderivaten, welche an beiden Stickstoffatomen des Antrachinonrings jeweils einen Aminoalkylaminosubstituenten tragen. Die im Rahmen dieses Verfahrens hergestellten Farbstoffe sollen eine hohe Affinität zu Keratinfasern und gute Waschechtheiten besitzen. Der mit den Farbstoffen erzielte Nuancenbereich reicht von gelb bis blau. Blaue Farbtöne mit einem Shift ins Graue lassen sich auch mit diesen Derivaten nicht erzielen. FR 1326406 A und US 3597254 offenbaren 1,4-Diamino-Anthrachinonfarbstoffe, die durch bestimmte aliphatische Reste subsituiert sind.

US 3576587 beschreibt Anthrachinone mit Amino- und Hydroxy-Substitution.

EP 2263642 A2 adressiert Blondiermittel, die durch Vermischen einer Oxidationsmittelzubereitung (A), einem Blondierpulver (B) und einer Färbezubereitung (C) erhalten werden. In der Färbezubereitung (C) können verschiedene direktziehende Farbstoffe enthalten sein.

WO 2011/160864 A2 betrifft Bleichmittel mit Ammoniumsalzen.

US 4310666 offenbart 1,4-Bis-subsituierte Aminoalkyl Amino-Anthrachinone als neue Farbstoffe.

Das Journal of Chromatography, 187 (1980) 212-217 beschreibt Methoden zur Reinheitsbestimmung von Anthrachinonfarbstoffen mittels HPLC.

Die in GB 2004293 A beschriebenen Anthrachinonfarbstoffe werden zur Reduktion des Tumorwachstums eingesetzt.

JP 193430 beschreibt Anthrachinonfarbstoffe als Dispersfarbstoffe.

WO 95/13813 A1 schließlich betrifft den Einsatz von Anthrachinonverbindungen als chemotherapeutische Agenzien.

Ein erster Gegenstand der vorliegenden Erfindung ist ein Mittel zum Färben und/oder Mattieren von keratinischen Fasern, insbesondere menschlichen Haaren, enthaltend in einem kosmetischen Träger mindestens ein 1,4-Diaminoanthrachinonderivat der Formel (I), 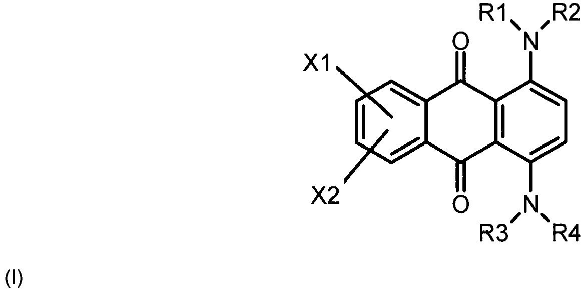 worin
R1, R3 jeweils für ein Wasserstoffatom stehen,
R2, R4 für identische Reste stehen und R2 und R4 jeweils für eine Gruppierung der Formel (II) stehen,

   -(CH₂)ₙ-Y1-(CH₂)ₘ-Y2 (II)

   worin
   Y1 für ein Sauerstoffatom (-O-) oder eine Gruppe -NR5- steht,
   Y2 für ein Wasserstoffatom, eine Hydroxygruppe, eine C₁-C₆-Alkoxygruppe, oder eine Gruppe-NR5R6 steht,
   n für die Zahl 3 steht,
   m für die Zahlen 2 oder 3 steht,
   R5, R6 unabhängig voneinander für ein Wasserstoffatom oder eine C₁-C₆-Alkylgruppe stehen, und
   X1, X2 unabhängig voneinander für ein Wasserstoffatom oder eine C₁-C₆-Alkoxygruppe stehen.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Der erfindungsgemäß verwendete Begriff "Färben von Keratinfasern" umfasst jedwede Form der Farbveränderung der Fasern. Umfasst sind insbesondere die unter den Begriffen Tönung, Blondierung, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise färbende Blondierungen. Unter dem Begriff "Mattierung von Keratinfasern" wird das Entgegenwirken von unerwünschten Nuancenverschiebungen verstanden, welche bei oxidativen Farbveränderungen von Keratinfasern, insbesondere bei Blondierungen oder bei Bleichprozessen, auftreten. Ziel ist der Mattierung ist die Abschwächung des durch unvollständige Blondierung hervorgerufenen orangen bis rötlichen Farbeindrucks und das Erzeugen einer silbrig-kühlen Farbwahrnehmung nach dem Blondierprozess. Die bei der Mattierung eingesetzten Wirkstoffe können in Form eines Nachbehandlungsschrittes nach dem Blondieren bzw. Bleichen der Keratinsfasern appliziert werden.

Es ist aber ebenso möglich, die für die Mattierung eingesetzten Wirkstoffe im Rahmen eines einstufigen Verfahrens zusammen mit dem Blondiermittel bzw. dem Bleichmittel auf die Keratinfasern aufzutragen. Als für die Mattierung geeignete Wirkstoffe können direktziehende Farbstoffe - entweder allein oder im Farbstoffgemisch - mit den geeigneten farblichen Eigenschaften verwendet werden. Darüber hinaus ist es ebenfalls möglich, für die Mattierung direktziehende Farbstoffe in Kombination mit Oxidationsfarbstoffvorprodukten (Entwicklern und Kupplern) einzusetzen. Die erfindungsgemäßen Mittel enthalten die Verbindungen der Formel (I) in einem kosmetischen Träger. Dieser kosmetische Träger ist bevorzugt wässrig, alkoholisch oder wässrig-alkoholisch. Zum Zwecke der Haarbehandlung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, zur Lagerung eine pulverförmige oder auch tablettenförmige Formulierung bereitzustellen. Diese wird dann vor der Anwendung in einem wässrigen Lösungsmittel oder mit organischen Lösungsmitteln bzw. mit Gemischen aus Wasser und organischen Lösungsmitteln unter Erhalt der Anwendungsmischung vermengt. Ein wässriger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Bevorzugte erfindungsgemäße Mittel sind dadurch gekennzeichnet, dass sie zusätzlich ein nichtwässriges Lösungsmittel enthalten, wobei bevorzugte erfindungsgemäße Mittel das Lösungsmittel in einer Konzentration von 0,1 bis 30 Gew.-%, bevorzugt in einer Konzentration von 1 bis 20 Gew.-%, ganz besonders bevorzugt in einer Konzentration von 2 bis 10 Gew.-%, jeweils bezogen auf das Mittel, enthalten. Die Substituenten R1 bis R6, X1 und X2 der Verbindung der Formel (I) sind nachfolgend beispielhaft erläutert: Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Beispiele für eine Polyhydroxy-C₂-C₆-alkylgruppe sind die 2,3-Dihydroxypropylgruppe, 3,4-Dihydroxybutylgruppe und die 2,4-Dihydroxybutylgruppe. Bevorzugte Beispiele für Cyano-C₁-C₆-alkylgruppen sind die Cyanomethylgruppe, die 2-Cyanoethylgruppe und die 3-Cyanopropylgruppe. Erfindungsgemäß bevorzugte Halogen-C₁-C₆-alkylgruppen sind die Chlormethylgruppe, die Brommethylgruppe, die Fluormethylgruppe, die 2-Chlorethylgruppe, die 2-Bromethylgruppe, die 2-Fluormethylgruppe, die 2-Chlorpropylgruppe, die 2-Brompropylgruppe, die 2-Fluropropylgruppe, die 3-Chlorpropylgruppe, die 3-Brompropylgruppe und die 3-Fluorpropylgruppe. Bevorzugte Beispiele von Aryl-C₁-C₆-alkylgruppen sind Benzyl, 1-Phenethyl und 2-Phenylethyl. Beispielhaft für Heteroaryl-C₁-C₆-alkylgruppen können die Imidazol-1-ylmethylgruppe, die Imidazol-2-ylmethylgruppe, die Imidazol-4-ylmethylgruppe, die Pyridin-2-ylgruppe, die Pyridin-3-ylgruppe und die Pyridin-4-ylmethylgruppe genannt werden. Erfindungsgemäß bevorzugte Arylgruppen sind die Phenylgruppe und die Naphthylgruppe. Beispiele für eine Heteroarylgruppe sind die Pyridin-2-ylgruppe, die Pyridin-3-ylgruppe, die Pyridin-4-ylgruppe, die Imidazol-1-ylgruppe, die Imidazol-2-ylgruppe und die Imidazol-4-ylgruppe. Beispielhaft für eine eine Amino-C₂-C₆-alkylgruppe können die 2-Aminoethylgruppe, die 2-Aminopropylgruppe, die 3-Aminopropylgruppe, die 2-Aminobutylgruppe, die 3-Aminobutylgruppe und die 4-Aminobutylgruppe genannt werden. Beispiele für eine C₁-C₆-Alkylamino-C₂-C₆-alkylgruppe sind die 2-(Methylamino)ethylgruppe, die 2-(Ethylamino)ethylgruppe, die 2-(Propylamino)ethylgruppe, die 3-(Methylamino)propylgruppe, die 3-(Ethylamino)propylgruppe, die 3-(Propylamino)propylgruppe, die 4-(Methylamino)butylgruppe, die 4-(Ethylamino)butylgruppe und die 4-(Propylamino)butylgruppe. Beispiele für eine C₁-C₆-Dialkylamino-C₂-C₆-alkylgruppe sind die 2-(Dimethylamino)ethylgruppe, die 2-(Diethylamino)ethylgruppe, die 2-(Dipropylamino)ethylgruppe, die 3-(Dimethylamino)propylgruppe, die 3-(Diethylamino)propylgruppe, die 3-(Dipropylamino)propylgruppe, die 4-(Dimethylamino)butylgruppe, die 4-(Diethylamino)butylgruppe und die 4-(Dipropylamino)butylgruppe. Erfindungsgemäß bevorzugte C₁-C₆-Alkoxygruppen sind die Methoxy- oder die Ethoxygruppe. Erfindungsgemäße Beispiele für eine Hydroxy-C₂-C₆-alkoxygruppe sind die 2-Hydroxyethoxygruppe, die 2-Hydroxypropoxygruppe (bzw. die 2-Hydroxypropyloxygruppe), die 3-Hydroxypropoxygruppe (bzw. die 3-Hydroxypropyloxygruppe), die 2-Hydroxybutoxygruppe (bzw. die 2-Hydroxybutyloxygruppe), die 3-Hydroxybutoxygruppe (bzw. die 3-Hydroxybutyloxygruppe) und die 4-Hydroxybutoxygruppe (bzw. die 4-Hydroxybutyloxygruppe). Als Beispiele für eine C₁-C₆-Alkylaminogruppe können Methylamino, Ethylamino, n-Propylamino, n-Butylamino und Isopropylamino genannt werden. Beispiele für die Di-C₁-C₆-alkylaminogruppe sind Dimethylamino, Diethylamino, Dipropylamino und Dibutylamino, wobei Dimethylamino und Diethylamino bevorzugt sind. Bevorzugte Beispiele für erfindungsgemäße C₁-C₆-Alkoxy-C₂-C₆-alkylgruppen sind die 2-(Methoxy)ethylgruppe, die 2-(Ethoxy)ethylgruppe, die 2-(Propoxy)ethylgruppe, die 3-(Methoxy)propylgruppe, die 3-(Ethoxy)-propylgruppe, die 3-(Propoxy)propylgruppe, die 4-(Methoxy)butylgruppe, die 4-(Ethoxy)butylgruppe und die 4-(Propoxy)butylgruppe. Erfindungsgemäße Beispiele für eine bevorzugte C₁-C₆-Alkoxy-C₂-C₆-alkyloxy-Gruppen sind die 2-Methoxy-ethoxygruppe, die 2-Methoxypropoxygruppe, die 3-Methoxypropoxygruppe, die 2-Methoxy-butoxygruppe, die 3-Methoxybutoxygruppe, die 4-Methoxy-butoxygruppe, 2-Ethoxy-ethoxygruppe, die 2-Ethoxypropoxygruppe, die 3-Ethoxypropoxygruppe, die 2-Ethoxy-butoxygruppe, die 3-Ethoxybutoxygruppe, die 4-Ethoxybutoxygruppe, 2-Propoxy-ethoxygruppe, die 2-Propoxypropoxygruppe, die 3-Propoxypropoxygruppe, die 2-Propoxy-butoxygruppe, die 3-Propoxybutoxygruppe und die 4-Propoxybutoxygruppe. Beispiele für Halogenatome sind F-, Cl-, Br- oder I-Atome, wobei Br- oder Cl-Atome ganz besonders bevorzugt sind.

Verbindungen der allgemeinen Formel (I) bei welchen die Substituenten X1 und X2 unabhängig voneinander ein Wasserstoffatom, eine Hydroxygruppe, eine C₁-C₆-Alkoxygruppe, eine C₁-C₆-Alkylgruppe, eine Aminogruppe oder ein Halogenatom darstellen, eignen sich besonders gut zur Mattierung und sind daher bevorzugt.

Der orange-stichigen Nuancenverschiebung von blondierten Keratinfasern lässt sich vor allem dann gut entgegenwirken, wenn hierzu Verbindungen der Formel (I) eingesetzt werden, bei welchen die Substituenten X1 und X2 unabhängig voneinander für ein Wasserstoffatom oder eine C₁-C₆-Alkoxygruppe stehen.

Eine erfindungsgemäßes Mittel zum Färben und/oder Mattieren von keratinischen Fasern ist dadurch gekennzeichnet, dass es mindestens eine Verbindung der Formel (I) enthält, bei der X1 und X2 unabhängig voneinander für ein Wasserstoffatom, oder eine C₁-C₆-Alkoxygruppe, eine C₁-C₆-Alkylgruppe, eine Aminogruppe oder ein Halogenatom, bevorzugt unabhängig voneinander für ein Wasserstoffatom oder eine C₁-C₆-Alkoxygruppe, stehen.

Ganz besonders bevorzugt ist es, wenn X1 und X2 beide für ein Wasserstoffatom stehen, oder aber wenn X1 für ein Wasserstoffatom und X2 für eine Methoxygruppe steht.

Verbindungen der Formel (I), bei welchen die Reste R1 und R3 für ein Wasserstoffatom, stehen, haben sich im Hinblick auf ihre anwendungstechnischen Eigenschaften ebenfalls als besonders geeignet herausgestellt.

Y1 steht für ein Sauerstoffatom (-O-) oder eine Gruppe -NR5-.

Die Reste R5 und R6 stehen unabhängig voneinander für ein Wasserstoffatom oder eine C₁-C₆-Alkylgruppe, ganz besonders bevorzugt unabhängig voneinander für ein Wasserstoffatom oder eine Methylgruppe.

Als besonders geeignet zur Lösung der erfindungsgemäßen Aufgabenstellung haben sich auch die Verbindungen der Formel (I) erwiesen, bei welchen Y2 für ein Wasserstoffatom, eine Hydroxygruppe, eine Methoxygruppe oder eine Aminogruppe (mit R5 und R6 gleich Wasserstoff) steht.

Im Zuge der zu dieser Erfindung führenden Arbeiten hat sich überraschenderweise herausgestellt, dass der Charakter der Formel (II) ganz entscheidenden Einfluß auf das Farbaufzugsvermögen und in diesem Zusammenhang auch auf die Echtheitseigenschaften der Farbstoffe besitzt. Die Reste R2 und R4 der allgemeinen Formel (I) stehen unabhängig voneinander zwingend jeweils für eine Gruppierung der Formel (II)

- (CH₂)ₙ-Y1-(CH₂)ₘ-Y2 (II)

wobei die Formel (II) jeweils zwei Alkylengruppen beinhaltet, deren Länge durch die Zahlen n und m definiert ist. Durch Optimierung der Kettenlängen kann die Affinität der Farbstoffe der Formel (I) zur Keratinfaser noch weiter gesteigert werden. Es hat sich herausgestellt, dass insbesondere dann intensive Färbungen mit guter Mattierleistung erhalten werden können, wenn n für die Zahl 3 steht. Vorteilhaft auf die Intensität der Färbungen wirkt sich ebenfalls aus, wenn m für die Zahlen 2 oder 3 steht.

Symmetrische Verbindungen der Formel (I) besitzen synthetisch die beste Zugänglichkeit. Damit sind symmetrische Verbindungen der Formel (I), bei denen die Reste R1 und R3 sowie die Reste R2 und R4 gleiche Substituenten darstellen, erfindungsgemäß.

Die Verbindungen der Formel (I) sind erfindungsgemäß, bei denen die Reste R1 und R3 jeweils für ein Wasserstoffatom stehen und die Reste R2 und R4 gleiche Substituenten darstellen.

Ein erfindungsgemäßes Mittel zum Färben und/oder Mattieren von keratinischen Fasern ist dadurch gekennzeichnet, dass es mindestens eine Verbindung der Formel (I) enthält, in der in der R1 und R3 jeweils für ein Wasserstoffatom stehen, R2 und R4 für identische Reste stehen und R2 und R4 jeweils für eine Gruppierung der Formel (II) stehen,

-(CH₂)n-Y1-(CH₂)m-Y2 (II)

worin
Y1 für ein Sauerstoffatom (-O-) oder eine Gruppe -NR5- steht,
Y2 für ein Wasserstoffatom, eine Hydroxygruppe, eine C₁-C₆-Alkoxygruppe oder eine Gruppe-NR5R6 steht,
n für die Zahl 3 steht,
m für die Zahlen 2 oder 3 steht, und
R5, R6 unabhängig voneinander für ein Wasserstoffatom oder eine C₁-C₆-Alkylgruppe stehen.

Eine weitere bevorzugte Ausführungsform sind Mittel zum Färben und/oder Mattieren von keratinischen Fasern, welche dadurch gekennzeichnet sind, dass sie mindestens eine Verbindung der allgemeinen Formel (I) enthalten, die ausgewählt ist aus
- 1,4-Bis[(3-ethoxypropyl)amino]-9,10-dihydroanthracen-9, 10-dion
- 1,4-Bis[(3-propoxypropyl)amino]-9,10-dihydroanthracen-9,10-dion
- 6-Methoxy-1,4-bis[(3-ethoxypropyl)amino]-9,10-dihydroanthracen-9,10-dion
- 6-Methoxy-1,4-bis[(3-propoxypropyl)amino]-9,10-dihydroanthracen-9,10-dion
- 1,4-Bis({[3-(2-hydroxyethoxy)propyl]amino})-9,10-dihydroanthracen-9,10-dion
- 1,4-Bis({[3-(3-hydroxypropoxy)propyl]amino})-9,10-dihydroanthracen-9,10-dion
- 6-Methoxy-1,4-bis({[3-(2-hydroxyethoxy)propyl]amino})-9,10-dihydroanthracen-9,10-dion
- 6-Methoxy-1,4-bis({[3-(3-hydroxypropoxy)propyl]amino})-9,10-dihydroanthracen-9,10-dion
- 1,4-Bis({3-[(2-hydroxyethyl)amino]propyl}amino)-9,10-dihydroanthracen-9,10-dion
- 1,4-bis({3-[(3-hydroxypropyl)amino]propyl}amino)-9,10-dihydroanthracen-9,10-dion
- 1,4-Bis({3-[(2-aminoethyl)amino]propyl}amino)-9,10-dihydroanthracen-9,10-dion
- 1,4-Bis({3-[(3-aminopropyl)amino]propyl}amino)-9,10-dihydroanthracen-9,10-dion Innerhalb dieser Gruppe sind die Verbindungen
- 1,4-Bis({[3-(2-hydroxyethoxy)propyl]amino})-9,10-dihydroanthracen-9,10-dion
- 1,4-Bis({[3-(3-hydroxypropoxy)propyl]amino})-9,10-dihydroanthracen-9,10-dion
- 1,4-Bis({3-[(2-hydroxyethyl)amino]propyl}amino)-9,10-dihydroanthracen-9,10-dion
- 1,4-bis({3-[(3-hydroxypropyl)amino]propyl}amino)-9,10-dihydroanthracen-9,10-dion
- 1,4-Bis({3-[(2-aminoethyl)amino]propyl}amino)-9,10-dihydroanthracen-9,10-dion und
- 1,4-Bis({3-[(3-aminopropyl)amino]propyl}amino)-9,10-dihydroanthracen-9,10-dion nochmals explizit ganz besonders bevorzugt, da sie das stärkste Farbaufzugsvermögen, die beste Mattierungsleistung und die beste Waschechtheit besitzen.

Die erfindungsgemäßen Mittel zum Färben und/oder Mattieren von Keratinfasern enthalten die Verbindung(en) der Formel (I) vorzugsweise in Mengen oberhalb von 1 ppm und unterhalb von 10 Gew.-%, jeweils bezogen auf das gesamte Mittel. Eine bevorzugte Ausführungsform ist dabei ein Mittel, welches die Verbindung(en) der Formel (I) in einem Anteil von 0,001 bis 5 Gew.-%, vorzugsweise von 0,0025 bis 3,5 Gew.-%, besonders bevorzugt von 0,005 bis 2,5 Gew.-% und insbesondere bevorzugt von 0,01 bis 1,5 Gew.-%, jeweils bezogen auf das Gesamtgewicht des Mittels, enthält.

In einer weiteren bevorzugten Ausführungsform enthalten die erfindungsgemäßen Mittel neben der Verbindung der Formel (I) zusätzlich mindestens einen weiteren direktziehenden Farbstoff. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen. Die zusätzlichen direktziehenden Farbstoffe werden jeweils bevorzugt in einem Anteil von 0,001 bis 2 Gew.-%, bezogen auf die gesamte Anwendungszubereitung, eingesetzt. Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC

Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4[(2-HyDroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol. Die Verbindungen der Formel (I) lassen sich in Kombination mit allen der oben genannten direktziehenden Farbstoffen in Mitteln zum Färben und/oder Mattieren von keratinischen Fasern einsetzen, wobei Färbeergebnisse mit guten Echtheitseigenschaften erhalten werden. Bestimmte Farbstoffkombinationen führen jedoch zu Färbungen mit besonders guter Haltbarkeit, so dass diese Kombinationen bevorzugt sind. Insbesondere bevorzugt ist es, wenn die Verbindung(en) der Formel (I) in Kombination mit Basic Yellow 57, Basic Red 76, Basic Brown 16 und Basic Brown 17 wie HC Blue 16 (Bluequat B), Basic Yellow 87, Basic Orange 31, Basic Red 51, HC Red 1, HC Red 3, HC Red 7, HC Red 10, HC Red 11, HC Red 13, HC Red BN, HC Blue 2, HC Blue 11, HC Blue 12, 2-Amino-4-nitrophenol, 1,4-Bis-(2-hydroxyethyl)-amino-2-nitrobenzol, 3-Nitro-4-(2-hydroxyethyl)aminophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 4-[(3-Hydroxypropyl)amino]-3-nitrophenol und/oder 4-Nitro-o-phenylendiamin eingesetzt werden. Die erfindungsgemäßen Mittel können weiterhin auch zusammen mit Oxidationsfärbemitteln eingesetzt werden. Solche Oxidationsfärbemittel enthalten zusätzlich mindestens ein Oxidationsfarbstoffvorprodukt, bevorzugt zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)-propyl]amin, N,4'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)-ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]-ethanol, 3-Amino-4-(2-methoxyethoxy)-5-methylphenylamin, 1-Amino-3-bis-(2-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Die zusätzlichen direktziehenden Farbstoffe, Entwicklerkomponenten und Kupplerkomponenten werden bevorzugt jeweils in einem Anteil von 0,0001 bis 5,0 Gew.-%, vorzugsweise 0,001 bis 3,5 Gew.-%, jeweils bezogen auf das anwendungsbereite Mittel, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können. Eine weitere bevorzugte Ausführungsform ist ein Mittel zum Färben und/oder Mattieren von keratinischen Fasern, welches dadurch gekennzeichnet ist, dass es zusätzlich - bezogen auf das Gewicht des gesamten Mittels -jeweils 0,001 bis 5 Gew.-% eines oder mehrerer Oxidationsfarbstoffvorprodukte und/oder direktziehender Farbstoffe enthält.

Soll die Mattierung mit den erfindungsgemäßen direktziehenden Farbstoffen und die oxidative Aufhellung der Keratinfasern in einem Schritt erfolgen, so enthalten die erfindungsgemäßen Mittel zusätzlich ein Oxidationsmittel, bevorzugt Wasserstoffperoxid und/oder eines seiner festen Anlagerungsprodukte an organische oder anorganische Verbindungen. In einer bevorzugten Ausführungsform wird Wasserstoffperoxid selbst als wässrige Lösung verwendet.

Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte anwendungsbereite Mittel des ersten Erfindungsgegenstands sind dadurch gekennzeichnet, dass sie, bezogen auf das Gesamtgewicht des anwendungsbereiten Mittels, 0,5 bis 20 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht des Mittels, enthalten. Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat. Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, in dem erfindungsgemäßen Mittel enthalten.Das Färbe- und/oder Mattier-Mittel kann zur Verstärkung der Blondierwirkung weitere Bleichkraftverstärker enthalten, wie beispielsweise Tetraacetylethylendiamin (TAED), 1,5-Diacetyl-2,4-dioxohexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluol-sulfonat, 2-Acetyl-1-methylpyridinium-p-toluolsulfonat, sowie N-Methyl-3,4-dihydroisochinolinium-p-toluolsulfonat.Zur weiteren Steigerung der Aufhellung kann der erfindungsgemäßen Zusammensetzung zusätzlich mindestens eine SiO₂-Verbindung, wie Kieselsäure oder Silicate, insbesondere Wassergläser, zugesetzt sein. Es kann erfindungsgemäß bevorzugt sein, die SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder. Die anwendungsbereiten Färbemittel können weiterhin zusätzliche Wirk-, Hilfs- und Zusatzstoffe enthalten, um die Färbeleistung zu verbessern und weitere gewünschte Eigenschaften der Mittel einzustellen. Die anwendungsbereiten Färbemittel werden als flüssige Zubereitung bereitgestellt und den Mitteln daher zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen, zwitterionischen, amphoteren und nichtionischen Tensiden und Emulgatoren ausgewählt.

Erfindungsgemäß bevorzugte Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugte Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß bevorzugte Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Erfindungsgemäß geeignete Mittel können auch kationische Tenside vom Typ der quartären Ammoniumverbindungen, der Esterquats und der Amidoamine enthalten. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus der Amidoamine stellt das unter der Bezeichnung Tegoamid® S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß verwendeten Mitteln bevorzugt in Anteilen von 0,05 bis 10 Gew.-%, bezogen auf das gesamte Mittel, enthalten.

Die anwendungsbereiten Färbemittel können weitere Hilfs- und Zusatzstoffe enthalten. So hat es sich als vorteilhaft erwiesen, wenn die Mittel mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind
- anionische, synthetische Polymere;
- kationische, synthetische Polymere;
- natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels zwischen 7 und 11, insbesondere zwischen 8 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Die erfindungsgemäßen Mittel eignen sich hervorragend zur Erzeugung von intensiven blauen, insbesondere zur Erzeugung von intensiven blauen bis grauen Nuancen auf keratinischen Fasern. In Verbindung mit ihren guten Echtheitseigenschaften und guter Stabilität gegenüber Oxidationsmitteln wie Wasserstoffperoxid sind sie zur Mattierung von Keratinfasern daher sehr gut geeignet.

Ein weiterer Gegenstand der vorliegenden Erfindung ist daher die kosmetische Verwendung eines Mittels des ersten Erfindungsgegenstands zur Erzeugung von intensiven Blaunuancen auf Keratinfasern und/oder zur Mattierung von Keratinfasern während oder nach einer oxidativen Aufhellung.

Die Mittel des ersten Erfindungsgegenstands können in Verfahren zum Mattieren menschlicher Haare genutzt werden. In einer ersten Ausführungsform des Verfahrens werden die erfindungsgemäßen Farbstoffe der Formel (I) nach dem Blondiervorgang in Form eines Nachbehandlungsmittels auf der Keratinfaser angewendet, wobei es sich bei dem Nachbehandlungsmittel um ein Shampoo, ein Gel, einen Conditioner, eine Creme, eine schäumende Lösung, einen Schaum oder ein Aerosol mit den üblichen zuvor beschriebenen weiteren Inhaltsstoffen handeln kann. Der Zeitraum zwischen dem Blondier- bzw. Bleichvorgang und der Anwendung des Nachbehandlungsmittels kann einige Tage bis Wochen betragen. Bevorzugt erfolgen der Blondiervorgang und die Mattierung jedoch innerhalb desselben Tages, insbesondere innerhalb einiger Stunden. In einer besonders bevorzugten Ausführungsform wird die Nachbehandlung direkt im Anschluß an den Blondiervorgang durchgeführt.

Bei den Blondiermitteln handelt es sich üblicherweise um Zweikomponentenprodukte, bei welchen eine oxidationsmittelhaltige Komponente (A1) kurz vor der Anwendung mit einem (Alkalisierungs-)Mittel (A2) vermischt und diese anwendungsbereite Mischung auf die Haare aufgetragen wird.

Von der Erfindung umfasst ist damit ein erstes Verfahren zum Mattieren von menschlichen Haaren, bei welchem unmittelbar vor der Anwendung
- ein kosmetisches Mittel (A1) zur oxidativen Aufhellung enthaltend 0,5 bis 20 Gew.-%, Wasserstoffperoxid mit
   - einem Mittel (A2) vermischt wird,
   - dieses anwendungsbereite Mittel auf die Keratinfasern aufgetragen, für einen Zeitraum von 5 bis 60 Minuten, bevorzugt von 15 bis 45 Minuten, auf den Keratinfasern belassen und dann wieder ausgespült wird,
   - die Keratinfasern gegebenenfalls getrocknet werden, und
- danach ein Nachbehandlungsmittel (B) auf die Keratinfasern aufgetragen, für einen Zeitraum von 2 bis 45 Minuten, bevorzugt 5 bis 30 Minuten, auf den Keratinfasern belassen und dann wieder ausgespült wird,
wobei es sich bei dem Mittel (B) um ein Mittel des ersten Erfindungsgegenstandes handelt.

In einer weiteren Ausführungsform können das Blondieren bzw. Bleichen der Haare und der Mattiervorgang in einem Schritt erfolgen, so dass das Mattierungsmittel zusammen mit dem oxidativen Aufhellmittel auf die Keratinfasern aufgetragen wird.

In diesem Fall handelt es sich bei dem Blondiermittel bevorzugt um ein Zweikomponentenprodukt, bei welchem eine oxidationsmittelhaltige Komponente (A1) kurz vor der Anwendung mit einem (Alkalisierungs-)Mittel (A2) vermischt wird, wobei das Mittel (A2) zusätzlich eine erfindungsgemäße Verbindung der Formel (I) enthält.

Damit ist auch ein zweites Verfahren erfindungsgemäß, bei welchem unmittelbar vor der Anwendung
- ein kosmetisches Mittel (A1) zur oxidativen Aufhellung enthaltend 0,5 bis 20 Gew.-%, Wasserstoffperoxid mit
   - einem Mittel (A2) vermischt wird,
   - dieses anwendungsbereite Mittel auf die Keratinfasern aufgetragen, für einen Zeitraum von 5 bis 60 Minuten, bevorzugt von 15 bis 45 Minuten, auf den Keratinfasern belassen und dann wieder ausgespült wird, und
- danach ein Nachbehandlungsmittel (B) auf die Keratinfasern aufgetragen, für einen Zeitraum von 2 bis 45 Minuten, bevorzugt 5 bis 30 Minuten, auf den Keratinfasern belassen und dann wieder ausgespült wird,
wobei es sich bei dem Mittel (A2) um ein Mittel des ersten Erfindungsgegenstandes handelt

Zur Verstärkung der Mattierwirkung ist es ebenfalls denkbar, dass die erfindungsgemäßen Farbstoffe der Formel (I) sowohl während des Blondiervorgangs zusammen mit dem Oxidationsmittel als auch in Form einer Nachbehandlungsmittels auf den keratinischen Fasern angewendet werden.

Ein weiterer Gegenstand der vorliegenden Erfindung ist demzufolge ein Verfahren zur Mattierung von keratinischen Fasern, bei welchem unmittelbar vor der Anwendung
- ein kosmetisches Mittel (A1) zur oxidativen Aufhellung enthaltend 0,5 bis 20 Gew.-%, Wasserstoffperoxid mit
   - einem Mittel (A2) vermischt wird,
   - dieses anwendungsbereite Mittel auf die Keratinfasern aufgetragen, für einen Zeitraum von 5 bis 60 Minuten, bevorzugt von 15 bis 45 Minuten, auf den Keratinfasern belassen und dann wieder ausgespült wird, und
- danach ein Nachbehandlungsmittel (B) auf die Keratinfasern aufgetragen, für einen Zeitraum von 2 bis 45 Minuten, bevorzugt 5 bis 30 Minuten, auf den Keratinfasern belassen und dann wieder ausgespült wird,
wobei es sich bei dem Mittel (A2) und/oder bei dem Mittel (B) um ein Mittel des ersten Erfindungsgegenstandes handelt.

Während der Einwirkzeit der Mittel auf der Faser kann es vorteilhaft sein, den Aufhellvorgang oder Mattiervorgang durch Wärmezufuhr zu unterstützen. Die Wärmezufuhr kann durch eine externe Wärmequelle, wie z.B. warme Luft eines Warmluftgebläses, als auch, insbesondere bei einer Haaraufhellung am lebenden Probanden, durch die Körpertemperatur des Probanden erfolgen. Bei letzterer Möglichkeit wird üblicherweise die behandelte Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Aufhellmittel einen stark tensidhaltigen Träger besitzt.

Die erfindungsgemäßen Mittel können als Einkomponentenmittel (Mattier- und Aufhellmittel) oder als Mehrkomponentenmittel wie Zweikomponenten Mittel oder Dreikomponentenmittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Bezüglich weiterer bevorzugter Ausführungsformen der erfindungsgemäßen Verwendung und Verfahren gilt mutatis mutandis das zu den erfindungsgemäßen Mitteln Gesagte.

### Beispiele

### 1. Synthesebeispiele

### 1.1. Synthesebeispiel 1: Synthese von 1,4-Bis({2-[(2-hydroxyethyl)amino]ethyl}amino)-9,10-dihydroanthracen-9,10-dion (DZ 1)

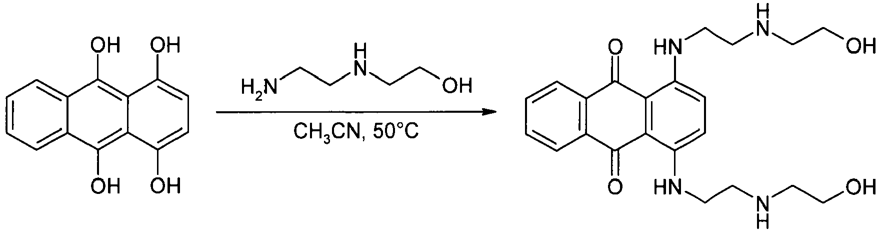

Zu einer Lösung von 10,0 g (40,0 mmol) Leukochinizarinin (9,10-Dihydroxy-2,3-dihydro-anthracen-1,4-dion) in 100 ml Acetonitril wurden 62,7 g (0,45 mol) 2-(2-Aminoethylamino)ethanol gegeben und das Reaktionsgemisch für 1 h bei 50°C unter Stickstoffatmosphäre gerührt. Anschließend wurde unter Rühren während 1 h bei 50°C Luft durch die Lösung geblasen. Danach wurde das Reaktionsgemisch mit 100 ml Acetonitril versetzt, auf 15°C gekühlt und der ausgefallene Feststoff abfiltriert. Dieser wurde mit reichlich Ethanol nachgewaschen und anschließend im Vakuum getrocknet. Man erhielt das 1,4-Bis({3-[(2-hydroxyethyl)amino]ethyl}amino)-9,10-dihydroanthracen-9,10-dion (DZ 1) als schwarzes Pulver (15,5 g, 37,0 mmol, 94%).
¹H-NMR (300 MHz, d₆-DMSO): δ = 2.65 (m, 4H, CH₂), 2.83 (m, 4H, CH₂), 3.49 (m, 8H, CH₂), 4.51 (br s, 2H, NH), 7.50 (s, 2H, Ar-H), 7.74 (m, 2H, Ar-H), 8.23 (m, 2H, Ar-H), 10.92 (m, 2H, NH)

### 1.2. Synthesebeispiel 2: 1,4-Bis({2-[(3-hydroxypropyl)amino]ethyl}amino)-9,10-dihydro-anthracen-9,10-dion (DZ 2)

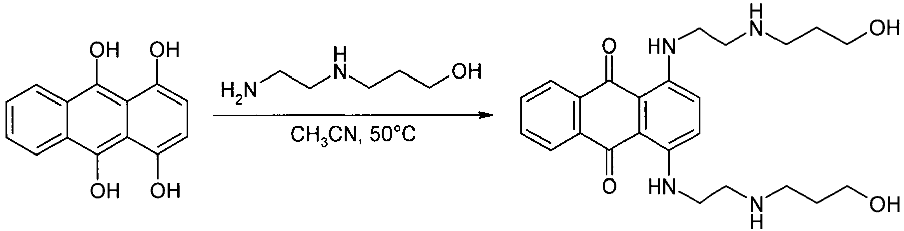

Zu einer Lösung von 10,0 g (40,0 mmol) Leukochinizarinin (9,10-Dihydroxy-2,3-dihydroanthra-cen-1,4-dion) in 100 ml Acetonitril wurde 48,0 g (0,41 mol) 3-(2-Aminoethylamino)propanol gegeben und das Reaktionsgemisch für 1 h bei 50°C unter Stickstoffatmosphäre gerührt. Dann wurde unter Rühren während 1 h bei 50°C Luft durch die Lösung geblasen. Danach wurde das Reaktionsgemisch mit 100 ml Acetonitril versetzt, auf 15°C gekühlt und der ausgefallene Feststoff abfiltriert. Dieser wurde mit reichlich Ethanol nachgewaschen und anschließend im Vakuum getrocknet. Man erhielt das 1,4-Bis({3-[(2-hydroxypropyl)amino]ethyl}amino)-9,10-dihydro-anthracen-9,10-dion (DZ 2) als dunkelblaues Pulver (3,3 g, 7,5 mmol, 19%).
¹H-NMR (300 MHz, d₆-DMSO): δ = 1.54 (m, 4H, CH₂), 2.67 (m, 4H, CH₂), 2.85 (m, 4H, CH₂), 3.50 (m, 8H, CH₂), 4.61 (br s, 4H, NH, OH), 7.47 (s, 2H, Ar-H), 7.72 (m, 2H, Ar-H), 8.21 (m, 2H, Ar-H), 10.92 (m, 2H, NH)

### 1.3. Synthesebeispiel 3: Synthese von 1,4-Bis({3-[(2-hydroxyethyl)amino]propyl}amino)-9,10-dihydroanthracen-9,10-dion (DZ 3)

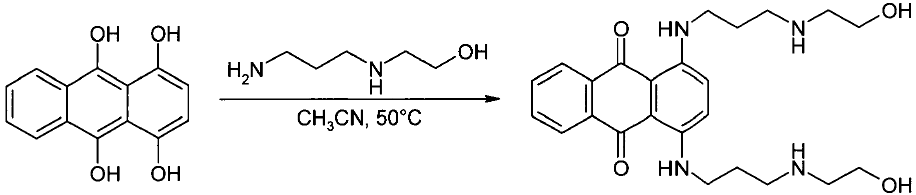

Zu einer Lösung von 10,0 g (40,0 mmol) Leukochinizarinin (9,10-Dihydroxy-2,3-dihydroanthra-cen-1,4-dion) in 100 ml Acetonitril wurde 27,0 g (0,23 mol) 2-(3-Aminopropylamino)ethanol gegeben und das Reaktionsgemisch für 1 h bei 50°C unter Stickstoffatmosphäre gerührt. Dann wurde unter Rühren während 1 h bei 50°C Luft durch die Lösung geblasen. Danach wurde das Reaktionsgemisch mit 100 ml Acetonitril versetzt, auf 15°C gekühlt und der ausgefallene Feststoff abfiltriert. Dieser wurde mit reichlich Ethanol nachgewaschen und anschließend im Vakuum getrocknet. Man erhielt das 1,4-Bis({3-[(2-hydroxyethyl)amino]propyl}amino)-9,10-dihydro-anthraoen-9,10-dion (DZ 3) als dunkelblaues Pulver (18,1 g, 7,5 mmol, 99%).
¹H-NMR (300 MHz, d₆-DMSO): δ = 1,74 (m, 4H, CH₂), 2.57 (m, 4H, CH₂), 2.63 (m, 4H, CH₂), 3.49 (m, 8H, CH₂), 7.42 (s, 2H, Ar-H), 7.74 (m, 2H, Ar-H), 8.22 (m, 2H, Ar-H), 10.87 (m, 2H, NH)

### 1.4. Synthesebeispiel 4: Synthese von 1,4-Bis({3-[(3-hydroxypropyl)amino]propyl}amino)-9,10-dihydroanthracen-9,10-dion (DZ 4)

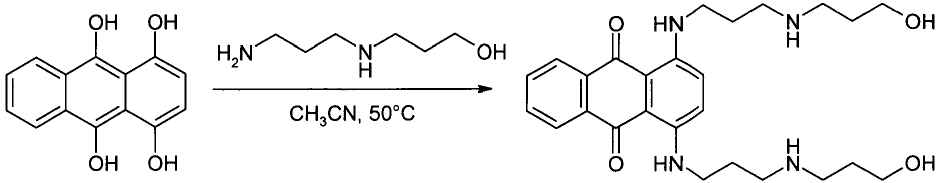

Zu einer Lösung von 10,0 g (40,0 mmol) Leukochinizarinin (9,10-Dihydroxy-2,3-dihydroanthracen-1,4-dion) in 100 ml Acetonitril wurde 60,0 g (0,45 mol) 3-(3-Aminopropylamino)propanol gegeben und das Reaktionsgemisch für 1 h bei 50°C unter Stickstoffatmosphäre gerührt. Anschließend wurde unter Rühren während 1 h bei 50°C Luft durch die Lösung geblasen. Danach wurde das Reaktionsgemisch mit 100 ml Acetonitril versetzt, auf 15°C gekühlt und der ausgefallene Feststoff abfiltriert. Dieser wurde mit reichlich Ethanol nachgewaschen und anschließend im Vakuum getrocknet. Man erhielt das 1,4-Bis({3-[(3-hydroxypropyl)amino]propyl}amino)-9,10-dihydro-anthracen-9,10-dion (**DZ 4**) als dunkelblaues Pulver (17,1 g, 7,5 mmol, 91%).
¹H-NMR (300 MHz, d₆-DMSO): δ = 1,750 (m, 4H, CH₂), 1,72 (m, 4H, CH₂), 2.50 (m, 4H, CH₂), 2.68 (m, 4H, CH₂), 3.41 (m, 8H, CH₂), 7.41 (s, 2H, Ar-H), 7.78 (m, 2H, Ar-H), 8.24 (m, 2H, Ar-H), 10.82 (m, 2H, NH)

### 1.5. Synthesebeispiel 5: Synthese von 1,4-Bis({2-[(2-aminoethyl)amino]ethyl}amino)-9,10-dihydroanthracen-9,10-dion (DZ 5)

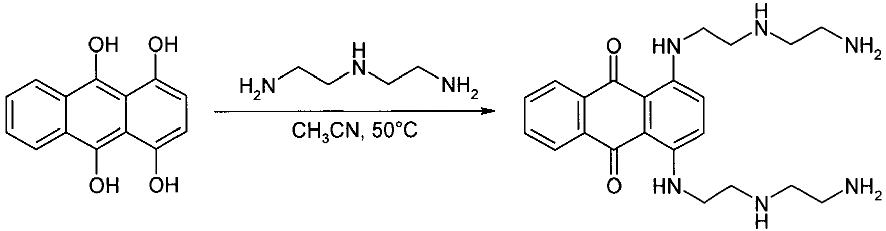

Zu einer Lösung von 10,0 g (40,0 mmol) Leukochinizarinin (9,10-Dihydroxy-2,3-dihydroanthra-cen-1,4-dion) in 100 ml Acetonitril wurden 46,1 g (0,45 mol) Diethylentriamin gegeben und das Reaktionsgemisch für 1 h bei 50°C unter Stickstoffatmosphäre gerührt. Anschließend wurde unter Rühren während 1 h bei 50°C Luft durch die Lösung geblasen. Danach wurde das Reaktionsgemisch mit 100 ml Acetonitril versetzt, auf 15°C gekühlt und der ausgefallene Feststoff abfiltriert. Dieser wurde mit reichlich Ethanol nachgewaschen und anschließend im Vakuum getrocknet. Man erhielt das 1,4-Bis({3-[(2-aminoethyl)amino]ethyl}amino)-9,10-dihydroanthracen-9,10-dion (DZ 5) als tiefblaue Pulver (4,4 g, 10,7 mmol, 27%).
¹H-NMR (300 MHz, d₆-DMSO): δ = 2.47 (m, 8H, CH₂), 2.53 (m, 8H, CH₂), 7.39 (s, 2H, Ar-H), 7.72 (m, 2H, Ar-H), 8.19 (m, 2H, Ar-H), 10.88 (m, 2H, NH)

### 2. Färbebeispiele

### 2.1. Herstellung der Färbecremes

Es wurden die folgenden Färbecremes hergestellt:

### 2.1.1 Färbecreme 1 (nichtionische Färbecreme)

| | |
|---|---|
| Cetearyl Alcohol | 6,0 g |
| Coconut Alcohol | 6,0 g |
| PEG-40 Hydrogenated Castor Oil | 1,0 g |
| Ceteareth-12 | 3,0 g |
| Ceteareth-20 | 3,0 g |
| PHB-Methylester | 0,3 g |
| PHB-Propylester | 0,2 g |
| Phenoxyethanol | 1,0 g |
| PEG-8 | 5,0 g |
| erfindungsgemäßer DZ | 1,0 g |
| Ammoniumsulfat | 1,0 g (in 30,0 g Wasser) |
| Hydroxyethylcellulose | 1,0 g (in 15,0 g Wasser) |
| NaOH 0,1% | ad pH-Wert |
| Wasser | ad 100 g |

Die ersten neun Komponenten wurden zusammen bei 80 °C aufgeschmolzen. Diese Mischung wurde mit einer wässrigen Lösung des Direktziehers und des Ammoniumsulfats in 30 g Wasser emulgiert. Anschließend wurde eine Quellung von 1,0 g Natrosol 250 HR in 15,0 g Wasser hinzugegeben. Der angegebene pH-Wert wurde mit 0,1% Natronlauge eingestellt, anschließend wurde mit Wasser auf 100 g aufgefüllt.

### 2.1.2 Färbecreme 2 (kationische Färbecreme)

| | |
|---|---|
| Cetearyl Alcohol | 4,0 g |
| Ceteareth-12 | 1,0 g |
| Dehyquart® A-CA | 2,0 g |
| Ammoniumsulfat | 1,0 g (in 30,0 g Wasser) |
| erfindungsgemäßer DZ | 1,0 g |
| Wasser | at 100 g |

Die ersten drei Komponenten wurden zusammen aufgeschmolzen, danach wurde die Schmelze mit heißem Wasser emulgiert. Dann wurden der in Wasser vorgelöste bzw. vordispergierte Farbstoff sowie die wässrige Ammoniumsulfatlösung hinzugegeben. Der pH-Wert wurde mit Ammoniak auf den angegebenen Wert eingestellt, anschließend wurde mit Wasser wurde auf 100 g aufgefüllt.

### 2.1.3 Färbecreme 3 (anionische Färbecreme)

| | |
|---|---|
| Cetearyl Alcohol | 1,0 g |
| Coconut Alcohol | 1,0 g |
| Akypo Soft® RLM 45N | 1,1 g |
| PHB-Propylester | 0,1 g |
| PHB-Methylester | 0,1 q |
| Ammoniumsulfat | 1,0 g (in 30,0 g Wasser) |
| erfindungsgemäßer DZ | 1,0 g |
| Wasser | ad 100 q |

Die ersten fünf Komponenten wurden zusammen aufgeschmolzen. Diese Schmelze wurde mit heißem Wasser emulgiert, anschließend wurde der in Wasser vorgelöste bzw. vordispergierte Farbstoff hinzu gegeben sowie die Ammoniumsulfatlösung hinzugefügt. Der angegebene pH-Wert wurde mit Ammoniak eingestellt, danach wurde mit Wasser auf 100 g aufgefüllt.

### 2.2 Verzeichnis der eingesetzten Rohstoffe

Akypo Soft RLM 45 NV®Laurylalkohol-4.5-EO-Essigsäure-Natrium-Salz (mind. 22% Aktivsubstanzgehalt; INCI-Bezeichnung: Sodium Laureth-5 Carboxylate)
Dehyquart® A-CA Trimethylhexadecylammoniumchlorid (ca. 24 -26 % Aktivsubstanz; INCI-Bezeichnung: Aqua (Water), Cetrimonium Chloride)

### 2.3. Ausfärbungen

Jeweils 1,8 g der Färbecreme wurden auf eine ca. 6 cm lange Strähne Menschenhaares (Kerling Euronaturhaar, blond) aufgetragen und dort 30 Minuten bei 30 °C belassen. Nach Beendigung der Einwirkzeit wurde das Haar ausgespült, mit einem üblichen Haarwaschmittel gewaschen und anschließend getrocknet. Die Haarsträhnen wurden in den nachfolgend angegebenen Nuancen gefärbt.
DZ 1: 1,4-Bis({2-[(2-hydroxyethyl)amino]ethyl}amino)-9,10-dihydroanthracen-9,10-dion
DZ 2: 1,4-Bis({2-[(3-hydroxypropyl)amino]ethyl}amino)-9,10-dihydroanthracen-9,10-dion
DZ 3: 1,4-Bis({3-[(2-hydroxyethyl)amino]propyl}amino)-9,10-dihydroanthracen-9,10-dion
DZ 4: 1,4-Bis({3-[(3-hydroxypropyl)amino]propyl}amino)-9,10-dihydroanthracen-9,10-dion
DZ 5: 1,4-Bis({2-[(2-aminoethyl)amino]ethyl}amino)-9,10-dihydroanthracen-9,10-dion

**Tabelle 1**

| Farbstoff | Färbecreme | pH-Wert | Farbnuance | Farbintensität |
|---|---|---|---|---|
| DZ 1 | 1 | 9,5 | gewitterblau (graublau) | ++ |
| DZ 1 | 2 | 9,5 | gewitterblau (graublau) | ++ |
| DZ 1 | 3 | 9,5 | gewitterblau (graublau) | + |
| DZ 2 | 1 | 9,5 | gräulich mattblau | ++ |
| DZ 2 | 2 | 9,5 | graublau | ++ |
| DZ 2 | 3 | 9,5 | kopenhagenerblau | ++ |
| DZ 3 | 1 | 9,5 | emailblau | +++ |
| DZ 3 | 2 | 9,5 | graublau | ++ |
| DZ 3 | 3 | 9,5 | graublau | +++ |
| DZ 4 | 1 | 9,5 | graublau | ++ |
| DZ 4 | 2 | 9,5 | tiefblau | +++ |
| DZ 4 | 3 | 9,5 | lapisblau | +++ |
| DZ 5 | 1 | 9,5 | mattblau - gewitterblau | + |
| DZ 5 | 2 | 9,5 | gewitterblau (graublau) | ++ |
| DZ 5 | 3 | 9,5 | dunkelblau | ++ |

| | | | | |
|---|---|---|---|---|
| Farbintensität: + = deutlich sichtbar ++ = gut +++ = sehr gut | | | | |

## Patentansprüche

1. Mittel zum Färben und/oder Mattieren von keratinischen Fasern, insbesondere menschlichen Haaren, enthaltend in einem kosmetischen Träger mindestens ein 1,4-Diaminoanthrachinonderivat der Formel (I), 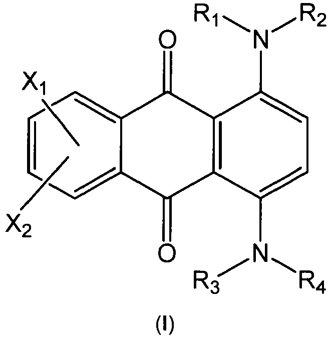 worin
R1 und R3 jeweils für ein Wasserstoffatom stehen,
R2 und R4 für identische Reste stehen und R2 und R4 jeweils für eine Gruppierung der Formel (II) stehen,
-(CH₂)nY₁-(CH₂)m-Y₂ (II)
worin,
Y1 für ein Sauerstoffatom (-O-) oder eine Gruppe -NR5- steht,
Y2 für ein Wasserstoffatom, eine Hydroxygruppe, eine C₁-C₆-Alkoxygruppe oder eine Gruppe -NR5R6 steht,
n für die Zahl 3 steht,
m für die Zahlen 2 oder 3 steht, und
R5, R6 unabhängig voneinander für ein Wasserstoffatom oder eine C₁-C₆-Alkylgruppe stehen,
X1, X2 unabhängig voneinander für ein Wasserstoffatom oder eine C₁-C₆-Alkoxygruppe, stehen,
**dadurch gekennzeichnet, dass** es eine oberflächenaktive Substanz ausgewählt aus anionischen, kationischen, zwitterionischen, amphoteren und nichtionischen Tensiden und Emulgatoren enthält.

2. Mittel nach Anspruch 1, **dadurch gekennzeichnet, dass** es mindestens eine Verbindung der Formel (I) enthält, die ausgewählt ist aus
- 1,4-Bis[(3-ethoxypropyl)amino]-9,10-dihydroanthracen-9,10-dion
- 1,4-Bis[(3-propoxypropyl)amino]-9,10-dihydroanthracen-9,10-dion
- 6-Methoxy-1,4-bis[(3-ethoxypropyl)amino]-9,10-dihydroanthracen-9,10-dion
- 6-Methoxy-1,4-bis[(3-propoxypropyl)amino]-9,10-dihydroanthracen-9,10-dion
- 1,4-Bis({[3-(2-hydroxyethoxy)propyl]amino})-9,10-dihydroanthracen-9,10-dion
- 1,4-Bis({[3-(3-hydroxypropoxy)propyl]amino})-9,10-dihydroanthracen-9,10-dion
- 6-Methoxy-1,4-bis({[3-(2-hydroxyethoxy)propyl]amino})-9,10-dihydroanthracen-9,10-dion
- 6-Methoxy-1,4-bis({[3-(3-hydroxypropoxy)propyl]amino})-9,10-dihydroanthracen-9,10-dion
- 1,4-Bis({3-[(2-hydroxyethyl)amino]propyl}amino)-9,10-dihydroanthracen-9,10-dion
- 1,4-bis({3-[(3-hydroxypropyl)amino]propyl}amino)-9,10-dihydroanthracen-9,10-dion
- 1,4-Bis({3-[(2-aminoethyl)amino]propyl}amino)-9,10-dihydroanthracen-9,10-dion
- 1,4-Bis({3-[(3-aminopropyl)amino]propyl}amino)-9,10-dihydroanthracen-9,10-dion

3. Mittel nach einem der Ansprüche 1 bis 2, **dadurch gekennzeichnet, dass** es die Verbindung(en) der Formel (I) in einem Anteil von 0,001 bis 5 Gew.-%, vorzugsweise von 0,0025 bis 3,5 Gew.-%, besonders bevorzugt von 0,005 bis 2,5 Gew.-% und insbesondere bevorzugt von 0,01 bis 1,5 Gew.-%, jeweils bezogen auf das Gesamtgewicht des Mittels, enthält.

4. Mittel nach einem der Ansprüche 1 bis 3, **dadurch gekennzeichnet, dass** es zusätzlich jeweils 0,001 bis 5 Gew.-% eines oder mehrerer Oxidationsfarbstoffvorprodukte und/oder direktziehender Farbstoffe enthält.

5. Mittel nach einem der Ansprüche 1 bis 4, **dadurch gekennzeichnet, dass** es mindestens eine Verbindung der Formel (I) ausgewählt aus
- 1,4-Bis({[3-(2-hydroxyethoxy)propyl]amino})-9,10-dihydroanthracen-9,10-dion
- 1,4-Bis({[3-(3-hydroxypropoxy)propyl]amino})-9,10-dihydroanthracen-9,10-dion
- 1,4-Bis({3-[(2-hydroxyethyl)amino]propyl}amino)-9,10-dihydroanthracen-9,10-dion
- 1,4-bis({3-[(3-hydroxypropyl)amino]propyl}amino)-9,10-dihydroanthracen-9,10-dion
- 1,4-Bis({3-[(2-aminoethyl)amino]propyl}amino)-9,10-dihydroanthracen-9,10-dion und
- 1,4-Bis({3-[(3-aminopropyl)amino]propyl}amino)-9,10-dihydroanthracen-9,10-dion und zusätzlich mindestens einen weiteren direktziehenden Farbstoff aus der Gruppe Basic Blue 7, Basic Blue 26, Basic Violet 2, Basic Violet 14, Basic Blue 99 und HC Blue 16 (Bluequat B) enthält.

6. Mittel nach einem der Ansprüche 1 bis 5, **dadurch gekennzeichnet, dass** es mindestens eine Verbindung der Formel (I) ausgewählt aus
- 1,4-Bis({[3-(2-hydroxyethoxy)propyl]amino})-9,10-dihydroanthracen-9,10-dion
- 1,4-Bis({[3-(3-hydroxypropoxy)propyl]amino})-9,10-dihydroanthracen-9,10-dion
- 1,4-Bis({3-[(2-hydroxyethyl)amino]propyl}amino)-9,10-dihydroanthracen-9,10-dion
- 1,4-bis({3-[(3-hydroxypropyl)amino]propyl}amino)-9,10-dihydroanthracen-9,10-dion
- 1,4-Bis({3-[(2-aminoethyl)amino]propyl}amino)-9,10-dihydroanthracen-9,10-dion und
- 1,4-Bis({3-[(3-aminopropyl)amino]propyl}amino)-9,10-dihydroanthracen-9,10-dion und zusätzlich mindestens einen weiteren direktziehenden Farbstoff aus der Gruppe HC Blue 2, HC Blue 11, HC Blue 12, Disperse Blue 3, HC Violet 1, Disperse Violet 1, Disperse Violet 4 und Disperse Black 9 enthält.

7. Mittel nach einem der Ansprüche 1 bis 6, **dadurch gekennzeichnet, dass** es einen pH-Wert zwischen 7 und 11 besitzt.

8. Mittel nach einem der Ansprüche 1 bis 7, **dadurch gekennzeichnet, dass** es mindestens ein Alkalisierungsmittel aus der Gruppe Ammoniak, Monoethanolamin, 2-Amino-2-methylpropanol und Triethanolamin enthält.

9. Mittel nach einem der Ansprüche 1 bis 8, **dadurch gekennzeichnet, dass** es zusätzlich ein oder mehrere kationische Tenside in einer Gesamtmenge von 0,05 bis 10 Gew.-%, bezogen auf das Gesamtgewicht des Mittels, enthält.

10. Kosmetische Verwendung eines Mittels nach einem der Ansprüche 1 bis 9 zur Erzeugung von intensiven Blaunuancen auf Keratinfasern und/oder zur Mattierung von Keratinfasern während oder nach einer oxidativen Aufhellung.

11. Verfahren zur Mattierung von keratinischen Fasern, bei welchem unmittelbar vor der Anwendung
- ein kosmetisches Mittel (A1) zur oxidativen Aufhellung enthaltend 0,5 bis 20 Gew.-%, Wasserstoffperoxid mit
- einem Mittel (A2) vermischt wird,
- dieses anwendungsbereite Mittel auf die Keratinfasern aufgetragen, für einen Zeitraum von 5 bis 60 Minuten, bevorzugt von 15 bis 45 Minuten, auf den Keratinfasern belassen und dann wieder ausgespült wird, und
- danach ein Nachbehandlungsmittel (B) auf die Keratinfasern aufgetragen, für einen Zeitraum von 2 bis 45 Minuten, bevorzugt 5 bis 30 Minuten, auf den Keratinfasern belassen und dann wieder ausgespült wird,
wobei es sich bei dem Mittel (A2) und/oder bei dem Mittel (B) um ein Mittel nach einem der Ansprüche 1 bis 9 handelt.

## Claims

1. An agent for dyeing and/or matting keratin fibers, in particular human hair, containing, in a cosmetic carrier, at least one 1,4-diaminoanthraquinone derivative of formula (I), 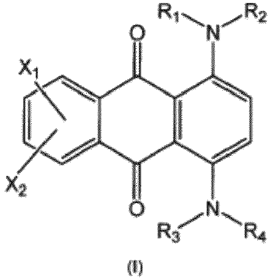 where
R1 and R3 both represent a hydrogen atom,
R2 and R4 represent identical functional groups and R2 and R4 both represent a group of formula (II),
-(CH₂)n-Y₁-(CH₂)m-Y₂ (II)
where
Y1 represents an oxygen atom (-O-) or an -NR5- group,
Y2 represents a hydrogen atom, a hydroxy group, a C₁-C₆ alkoxy group or an -NR5R6 group,
n represents the number 3,
m represents the numbers 2 or 3, and
R5 and R6 represent, independently of each other, a hydrogen atom or a C₁-C₆ alkyl group,
X1 and X2 represent, independently of each other, a hydrogen atom or a C₁-C₆ alkoxy group,
**characterized in that** said agent contains a surface-active substance selected from anionic, cationic, zwitterionic, amphoteric and non-ionic surfactants and emulsifiers.

2. The agent according to claim 1, **characterized in that** it contains at least one compound of formula (I), which is selected from
- 1,4-bis[(3-ethoxypropyl)amino]-9,10-dihydroanthracene-9,10-dione
- 1,4-bis[(3-propoxypropyl)amino]-9,10-dihydroanthracene-9,10-dione
- 6-methoxy-1,4-bis[(3-ethoxypropyl)amino]-9,10-dihydroanthracene-9,10-dione
- 6-methoxy-1,4-bis[(3-propoxypropyl)amino]-9,10-dihydroanthracene-9,10-dione
- 1,4-bis({[3-(2-hydroxyethoxy)propyl]amino})-9,10-dihydroanthracene-9,10-dione
- 1,4-bis({[3-(3-hydroxypropoxy)propyl]amino})-9,10-dihydroanthracene-9,10-dione
- 6-methoxy-1,4-bis({[3-(2-hydroxyethoxy)propyl]amino})-9,10-dihydroanthracene-9,10-dione
- 6-methoxy-1,4-bis({[3-(3-hydroxypropoxy)propyl]amino})-9,10-dihydroanthracene-9,10-dione
- 1,4-bis({3-[(2-hydroxyethyl)amino]propyl}amino)-9,10-dihydroanthracene-9,10-dione
- 1,4-bis({3-[(3-hydroxypropyl)amino]propyl}amino)-9,10-dihydroanthracene-9,10-dione
- 1,4-bis({3-[(2-aminoethyl)amino]propyl}amino)-9,10-dihydroanthracene-9,10-dione
- 1,4-bis({3-[(3-aminopropyl)amino]propyl}amino)-9,10-dihydroanthracene-9,10-dione

3. The agent according to one of claims 1 to 2, **characterized in that** it contains the compound(s) of formula (I) in a proportion of from 0.001 to 5 wt.%, preferably 0.0025 to 3.5 wt.%, particularly preferably 0.005 to 2.5 wt.%, and more particularly preferably 0.01 to 1.5 wt.%, in each case based on the total weight of the agent.

4. The agent according to one of claims 1 to 3, **characterized in that** it additionally contains in each case from 0.001 to 5 wt.% of one or more oxidation dye precursors and/or direct dyes.

5. The agent according to one of claims 1 to 4, **characterized in that** it contains at least one compound of formula (I) selected from
- 1,4-bis({[3-(2-hydroxyethoxy)propyl]amino})-9,10-dihydroanthracene-9,10-dione
- 1,4-bis({[3-(3-hydroxypropoxy)propyl]amino})-9,10-dihydroanthracene-9,10-dione
- 1,4-bis({3-[(2-hydroxyethyl)amino]propyl}amino)-9,10-dihydroanthracene-9,10-dione
- 1,4-bis({3-[(3-hydroxypropyl)amino]propyl}amino)-9,10-dihydroanthracene-9,10-dione
- 1,4-bis({3-[(2-aminoethyl)amino]propyl}amino)-9,10-dihydroanthracene-9,10-dione and
- 1,4-bis({3-[(3-aminopropyl)amino]propyl}amino)-9,10-dihydroanthracene-9,10-dione and additionally at least one additional direct dye from the group comprising Basic Blue 7, Basic Blue 26, Basic Violet 2, Basic Violet 14, Basic Blue 99 and HC Blue 16 (Bluequat B).

6. The agent according to one of claims 1 to 5, **characterized in that** it contains at least one compound of formula (I) selected from
- 1,4-bis({[3-(2-hydroxyethoxy)propyl]amino})-9,10-dihydroanthracene-9,10-dione
- 1,4-bis({[3-(3-hydroxypropoxy)propyl]amino})-9,10-dihydroanthracene-9,10-dione
- 1,4-bis({3-[(2-hydroxyethyl)amino]propyl}amino)-9,10-dihydroanthracene-9,10-dione
- 1,4-bis({3-[(3-hydroxypropyl)amino]propyl}amino)-9,10-dihydroanthracene-9,10-dione
- 1,4-bis({3-[(2-aminoethyl)amino]propyl}amino)-9,10-dihydroanthracene-9,10-dione and
- 1,4-bis({3-[(3-aminopropyl)amino]propyl}amino)-9,10-dihydroanthracene-9,10-dione and additionally at least one additional direct dye from the group comprising HC Blue 2, HC Blue 11, HC Blue 12, Disperse Blue 3, HC Violet 1, Disperse Violet 1, Disperse Violet 4 and Disperse Black 9.

7. The agent according to one of claims 1 to 6, **characterized in that** it has a pH of between 7 and 11.

8. The agent according to one of claims 1 to 7, **characterized in that** it contains at least one alkalizing agent from the group comprising ammonia, monoethanolamine, 2-amino-2-methylpropanol and triethanolamine.

9. The agent according to one of claims 1 to 8, **characterized in that** it additionally contains one or more cationic surfactants in a total amount of from 0.05 to 10 wt.%, based on the total weight of the agent.

10. The cosmetic use of an agent according to one of claims 1 to 9 for producing intense shades of blue on keratin fibers and/or for matting keratin fibers during or after oxidative lightening.

11. A method for matting keratin fibers, in which, immediately before application,
- a cosmetic agent (A1) for oxidative lightening containing from 0.5 to 20 wt.% of hydrogen peroxide is mixed with
- an agent (A2),
- said ready-to-apply agent is applied to the keratin fibers, left on the keratin fibers for a period of from 5 to 60 minutes, preferably 15 to 45 minutes, and then rinsed out, and
- a post-treatment agent (B) is subsequently applied to the keratin fibers, left on the keratin fibers for a period of from 2 to 45 minutes, preferably 5 to 30 minutes, and then rinsed out,
wherein the agent (A2) and/or the agent (B) is an agent according to one of claims 1 to 9.

## Revendications

1. Agent de coloration et/ou de mise au mat de fibres kératiniques, en particulier de cheveux humains, contenant dans un support cosmétique au moins un dérivé de 1,4-diaminoanthraquinone de la formule (I), 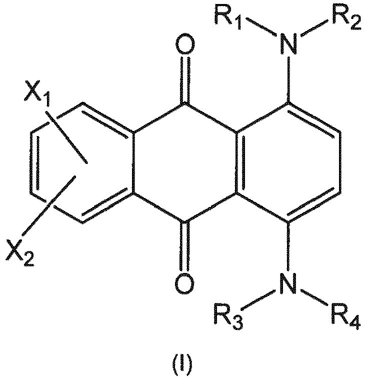 dans laquelle
R1 et R3 représentent respectivement un atome d'hydrogène,
R2 et R4 représentent des radicaux identiques et R2 et R4 représentent respectivement un groupement de la formule (II),
-(CH₂)n-Y₁-(CH₂)m-Y₂ (II)
dans laquelle
Y1 représente un atome d'oxygène (-O-) ou un groupe -NR5-,
Y2 représente un atome d'hydrogène, un groupe hydroxy, un groupe alkoxy en C₁-C₆ ou un groupe -NR5R6,
n représente le chiffre 3,
m représente les chiffres 2 ou 3, et
R5, R6 représentent indépendamment l'un de l'autre un atome d'hydrogène ou un groupe alkyle en C₁-C₆,
X1, X2 représentent indépendamment l'un de l'autre un atome d'hydrogène ou un groupe alkoxy en C₁-C₆,
**caractérisé en ce qu'**il contient une substance à effet tensioactif sélectionnée parmi des tensioactifs et émulsifiants cationiques, zwitterioniques, amphotères et non-ioniques.

2. Agent selon la revendication 1, **caractérisé en ce qu'**il contient au moins un composé de la formule (I), sélectionné parmi
- 1,4-bis[(3-éthoxypropyl)amino]-9,10-dihydroanthracène-9,10-dione
- 1,4-bis[(3-propoxypropyl)amino]-9,10-dihydroanthracène-9,10-dione
- 6-méthoxy-1,4-bis[(3-éthoxypropyl)amino]-9,10-dihydroanthracène-9,10-dione
- 6-méthoxy-1,4-bis[(3-propoxypropyl)amino]-9,10-dihydroanthracène-9,10-dione
- 1,4-bis({[3-(2-hydroxyéthoxy)propyl]amino})-9,10-dihydroanthracène-9,10-dione
- 1,4-bis({[3-(3-hydroxypropoxy)propyl]amino})-9,10-dihydroanthracène-9,10-dione
- 6-méthoxy-1,4-bis({[3-(2-hydroxyéthoxy)propyl]amino})-9,10-dihydroanthracène-9,10-dione
- 6-méthoxy-1,4-bis({[3-(3-hydroxypropoxy)propyl]amino})-9,10-dihydroanthracène-9,10-dione
- 1,4-bis({3-[(2-hydroxyéthyl)amino]propyl}amino)-9,10-dihydroanthracène-9,10-dione
- 1,4-bis({3-[(3-hydroxypropyl)amino]propyl}amino)-9,10-dihydroanthracène-9,10-dione
- 1,4-bis({3-[(2-aminoéthyl)amino]propyl}amino)-9,10-dihydroanthracène-9,10-dione
- 1,4-bis({3-[(3-aminopropyl)amino]propyl}amino)-9,10-dihydroanthracène-9,10-dione

3. Agent selon l'une des revendications 1 à 2, **caractérisé en ce qu'**il contient le(s) composé(s) de la formule (I) dans une part allant de 0,001 à 5 % en poids, de préférence de 0,0025 à 3,5 % en poids, de manière particulièrement préférée de 0,005 à 2,5 % en poids et en particulier de manière préférée de 0,01 à 1,5 % en poids, respectivement rapporté au poids total de l'agent.

4. Agent selon l'une des revendications 1 à 3, **caractérisé en ce qu'**il contient en outre respectivement de 0,001 à 5 % en poids d'un ou de plusieurs précurseurs de colorants par oxydation et/ou de colorants à action directe.

5. Agent selon l'une des revendications 1 à 4, **caractérisé en ce qu'**il contient au moins un composé de la formule (I) sélectionné parmi
- 1,4-bis({[3-(2-hydroxyéthoxy)propyl]amino})-9,10-dihydroanthracène-9,10-dione
- 1,4-bis({[3-(3-hydroxypropoxy)propyl]amino})-9,10-dihydroanthracène-9,10-dione
- 1,4-bis({3-[(2-hydroxyéthyl)amino]propyl}amino)-9,10-dihydroanthracène-9,10-dione
- 1,4-bis({3-[(3-hydroxypropyl)amino]propyl}amino)-9,10-dihydroanthracène-9,10-dione
- 1,4-bis({3-[(2-aminoéthyl)amino]propyl}amino)-9,10-dihydroanthracène-9,10-dione et
- 1,4-bis({3-[(3-aminopropyl)amino]propyl}amino)-9,10-dihydroanthracène-9,10-dione et en outre au moins un autre colorant à action directe issu du groupe comprenant Basic Blue 7, Basic Blue 26, Basic Violet 2, Basic Violet 14, Basic Blue 99 et HC Blue 16 (Bluequat).

6. Agent selon l'une des revendications 1 à 5, **caractérisé en ce qu'**il contient au moins un composé de la formule (I) sélectionné parmi
- 1,4-bis({[3-(2-hydroxyéthoxy)propyl]amino})-9,10-dihydroanthracène-9,10-dione
- 1,4-bis({[3-(3-hydroxypropoxy)propyl]amino})-9,10-dihydroanthracène-9,10-dione
- 1,4-bis({3-[(2-hydroxyéthyl)amino]propyl}amino)-9,10-dihydroanthracène-9,10-dione
- 1,4-bis({3-[(3-hydroxypropyl)amino]propyl}amino)-9,10-dihydroanthracène-9,10-dione
- 1,4-bis({3-[(2-aminoéthyl)amino]propyl}amino)-9,10-dihydroanthracène-9,10-dione et
- 1,4-bis({3-[(3-aminopropyl)amino]propyl}amino)-9,10-dihydroanthracène-9,10-dione et en outre au moins un autre colorant à action directe issu du groupe comprenant HC Blue 2, HC Blue 11, HC Blue 12, Disperse Blue 3, HC Violet 1, Disperse Violet 1, Disperse Violet 4 et Disperse Black 9.

7. Agent selon l'une des revendications 1 à 6, **caractérisé en ce qu'**il possède une valeur pH comprise entre 7 et 11.

8. Agent selon l'une des revendications 1 à 7, **caractérisé en ce qu'**il contient au moins un agent d'alcalinisation issu du groupe comprenant l'ammoniac, la monoéthanolamine, le 2-amino-2-méthylpropanol et la triéthanolamine.

9. Agent selon l'une des revendications 1 à 8, **caractérisé en ce qu'**il contient en outre un ou plusieurs tensioactifs cationiques dans une quantité totale allant de 0,05 à 10 % en poids, rapporté au poids total de l'agent.

10. Utilisation cosmétique d'un agent selon l'une des revendications 1 à 9 afin de créer des nuances de bleu intenses sur les fibres kératiniques et/ou pour la mise au mat de fibres kératiniques lors de ou après un éclaircissement par oxydation.

11. Procédé de mise au mat de fibres kératiniques, lors duquel, immédiatement avant l'utilisation
- un agent cosmétique (A1) pour l'éclaircissement par oxydation contenant 0,5 à 20 % en poids en peroxyde d'hydrogène est mélangé à
- un agent (A2),
- cet agent prêt à l'emploi est appliqué sur les fibres kératiniques, est laissé à reposer pour une durée allant de 5 à 60 minutes, de manière préférée de 15 à 45 minutes sur les fibres kératiniques, puis est de nouveau rincé, et
- suite à cela un agent de post traitement (B) est appliqué sur les fibres kératiniques, est laissé à reposer pour une durée allant de 2 à 45 minutes, de manière préférée de 5 à 30 minutes sur les fibres kératiniques, puis est de nouveau rincé,
l'agent (A2) et/ou l'agent (B) étant un agent selon l'une des revendications 1 à 9.
